# EUROPÄISCHE PATENTSCHRIFT

(11) **EP 0 543 249 B1**
(45) Veröffentlichungstag und Bekanntmachung des Hinweises auf die Patenterteilung: **10.04.1996**
(21) Anmeldenummer: 92119145.8
(22) Anmeldetag: 09.11.1992
(51) Int. Cl.: C07D 317/38

(54) **Verfahren zur Herstellung von Alkylencarbonaten**
Method for the preparation of alkylene carbonates
Méthode pour la préparation de carbonates d'alkylène

(30) Priorität: 22.11.1991 DE 4138438; 02.04.1992 DE 4210943
(43) Veröffentlichungstag der Anmeldung: 26.05.1993
(73) Patentinhaber: BAYER AG, D-51368 Leverkusen (DE)
(72) Erfinder: Mais, Franz-Josef, Dr., W-4000 Düsseldorf 1 (DE); Buysch, Hans-Josef, Dr., W-4150 Krefeld (DE); Mendoza-Frohn, Christine, Dr., W-4006 Erkrath 2 (DE); Klausener, Alexander, Dr., W-5190 Stolberg (DE)

(56) Entgegenhaltungen:
- GB-A- 2 011 402
- CHEMICAL ABSTRACTS, Band 79, Nr. 23, 10. Dezember 1973, Columbus, OH (US); F. OOTA et al., Seite 331, Nr. 136527r

## Beschreibung

Die vorliegende Erfindung betrifft ein Verfahren zur Herstellung von Alkylencarbonaten durch Umsetzung von Alkylenoxiden mit Kohlendioxid in Gegenwart von Katalysatoren. Als Katalysatoren werden Mischkatalysatoren der Formel

a[MX]/b[ZnY₂] (III),

eingesetzt, die Mischkatalysatoren aus Alkalimetallhalogeniden und Zinkhalogeniden darstellen. Diese Katalysatoren sind durch Halogenverbindungen zusätzlich aktivierbar.

Alkylencarbonate, beispielsweise Ethylencarbonat oder Propylencarbonat, sind wertvolle Zwischenprodukte für die Herstellung von Kunststoffen, wie beispielsweise Polycarbonaten, oder sie dienen als Oxalkylierungsmittel zur Synthese von Farbstoffen, Pflanzenschutzmitteln oder Pharmazeutika. Außerdem können sie als Lösungsmittel, beispielsweise in der Faserproduktion, dienen.

Es ist bereits bekannt, Alkylenoxide in Gegenwart von Katalysatoren mit Kohlendioxid zu den cyclischen Alkylencarbonaten umzusetzen. Dazu sind allerdings, um technisch ausreichende Reaktionsgeschwindigkeiten zu erzielen, hohe Temperaturen und hohe Drücke erforderlich. Dies ist insofern problematisch, da einerseits besonders die niedermolekularen Alkylencarbonate technisches Interesse beanspruchen, andererseits aber die zu ihrer Herstellung erforderlichen Alkylenoxide zur Zersetzung neigen und daher bei hohen Temperaturen und hohen Drücken besondere sicherheitstechnische Einrichtung erfordern. Hohe Temperaturen und hohe Drücke bedingen weiterhin einen hohen apparativen Aufwand, um die gewünschten Reaktionsbedingungen aufrechterhalten zu können. Als Katalysatoren sind bisher eine Vielzahl von Verbindungen bekannt geworden.

Solche Katalysatoren umfassen Ammonium-, Phosphonium- und Sulfonium-Salze (US 2.773.070; US 2.994.705; DE-OS 32 44 456), eine Kombination aus protischen Verbindungen und stickstoffhaltigen Basen (DE-OS 26 11 087), Arsoniumhalogenide (EP 180 387), tertiäre Phosphine (WO 84/03701), Stickstoffbasen (US 3.535.341; US 3.535.342) und Alkalihalogenide (BE 872 960). Aus der allgemeinen chemischen Literatur ist die Umsetzung von Alkylenoxiden mit Kohlendioxid in Gegenwart eines Katalysatorsystems aus einem Metallchlorid und Tetraalkylammoniumiodiden bekannt (Chem. Ber. 119 (1986), 1090, und Chem. Ber. 123 (1990), 277). Weiterhin ist die Umsetzung von Alkylenoxiden mit Kohlendioxid in Gegenwart von Katalysatorsystemen, bestehend aus einem Organozinnhalogenid und einem quartären Phosphoniumhalogenid, bekannt (Bull. Chem. Soc. Japan 60 (1987), 1552). Die in diesen Publikationen vorgeschlagene Verfahrensweise besteht darin, eine Lösung des Katalysators in dem Alkylenoxid mit Kohlendioxid zu sättigen und so bei Normaldruck und wenig erhöhter Temperatur die Umsetzung zum Alkylencarbonat durchzuführen. Wegen der beschriebenen Zersetzungsgefahr der Alkylenoxide ist diese Verfahrensweise aus sicherheitstechnischen Gründen bedenklich. Weiterhin sind die lange Reaktionszeit (5 Stunden und darüber) und die relativ hohe Aufwandmenge an Katalysator ungünstig. Ferner ist es ungünstig, daß diese Katalysatoren im allgemeinen, wie eigene Versuche zeigten (Vergleichsbeispiele 3 und 7), bei einer technisch anzustrebenden Rückführung bald ihre Aktivität verlieren und somit eine Entsorgung des inaktiven Altkatalysators in großer Menge notwendig wird.

Es wurde nunmehr ein Verfahren zur Herstellung von Alkylencarbonaten der Formel 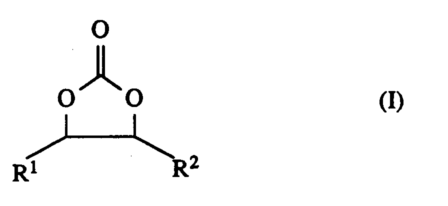
durch Umsetzung von Alkylenoxiden der Formel 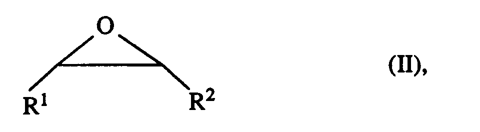
wobei in den Formeln
- R¹ und R²: unabhängig voneinander Wasserstoff, substituiertes oder nicht substituiertes C₁-C₄-Alkyl, substituiertes oder nicht substituiertes C₂-C₄-Alkenyl oder substituiertes oder nicht substituiertes C₆-C₁₂-Aryl bedeuten und
- R¹ und R²: gemeinsam mit den beiden Dreiring-C-Atomen einen gesättigten carbocyclischen Ring mit 5-8 Ringgliedern bedeuten können,
mit Kohlendioxid in Gegenwart von Katalysatoren gefunden, das dadurch gekennzeichnet ist, daß man aktivierbare Katalysatoren der Formel

a[MX]/b[ZnY₂] (III)

einsetzt, worin
- M: ein Alkalimetall, bevorzugt Li, Na oder K, besonders bevorzugt Na oder K, bedeutet,
- X und Y: unabhängig voneinander Chlor, Brom oder Iod bedeuten und
- a und b: gebrochene oder ganze Zahlen in einem Bereich von 0,001-2 bedeuten,
wobei die Aktivierung durchgeführt werden kann durch Zusatz einer Halogenverbindung der Formel

R³-Z (IV),

worin
- Z: für Chlor, Brom oder Iod steht und
- R³: Wasserstoff, Chlor, Brom oder Iod, den Rest eines anorganischen oder organischen Säurehalogenids, den Rest eines Benzyl-, Benzal- oder Benzotrihalogenids, C₄-C₈-tert.-Alkyl, Phenacyl, (Meth)Allyl oder -CHR¹-CHR²-OH, worin R¹ und R² die gegebene Bedeutung haben, bedeutet und für den Fall, daß Z für Iod steht, auch Cl₃ oder Br₃ bedeuten kann,
und daß man bei einer Reaktionstemperatur von 40 bis 250°C und einem molaren Verhältnis von Alkylenoxid zu CO₂ von 1:1-10 arbeitet.

C₁-C₄-Alkylreste können geradkettig oder verzweigt sein, sowie durch Halogen, Aryl oder Alkoxy substituiert sein, beispielsweise Methyl, Ethyl, Propyl, Isopropyl, Methoxymethyl, Chlormethyl, Benzyl und andere; bevorzugt ist nicht substituiertes C₁-C₄-Alkyl.

C₂-C₄-Alkenyl kann geradkettig oder verzweigt, sowie durch Halogen, Alkyl oder Aryl substituiert sein, beispielsweise Vinyl, Propenyl, 2-Phenyl-vinyl, 2-Chlor-vinyl und andere; bevorzugt ist Vinyl.

C₆-C₁₂-Aryl kann beispielsweise Phenyl, Naphthyl oder Biphenyl, bevorzugt Phenyl, sein. Solche Arylreste können durch Alkyl, Alkoxy, Halogen oder Nitro substituiert sein. Beispiele für substituiertes Aryl sind: p-Tolyl, o-Tolyl, p-Methoxyphenyl, p-Nitrophenyl, p-Chlorphenyl.

Als carbocyclischer Ring mit 5-8 Ringgliedern, der von R¹ und R² gemeinsam mit den beiden Dreiring-C-Atomen gebildet werden kann, sei beispielsweise genannt: der Cyclopentanring, der Cyclohexanring oder der Cyclooctanring.

In bevorzugter Weise werden im erfindungsgemäßen Verfahren Alkylenoxide eingesetzt, in denen die Reste R¹ und R² unabhängig voneinander Wasserstoff, Methyl, Ethyl, Propyl, Phenyl oder Chlormethyl bedeuten.

Als Alkalimetall M sei Lithium, Natrium, Kalium, Rubidium und Caesium, bevorzugt Lithium, Natrium und Kalium, besonders bevorzugt Natrium und Kalium, genannt.

Als Halogene X, Y und Z stehen unabhängig voneinander Chlor, Brom oder Iod. Bevorzugt steht für X und Z Brom, oder Iod, besonders bevorzugt Brom. Bevorzugt steht für Y Chlor oder Brom, besonders bevorzugt Brom.

Es ist erfindungsgemäß bevorzugt, daß mindestens eines der Halogene X und Y Brom bedeutet; in besonders bevorzugter Weise bedeuten sowohl X als auch Y Brom. In einer ganz besonders bevorzugten Weise bedeuten alle drei Halogene X, Y und Z Brom.

Als Halogenverbindung der Formel (IV) können grundsätzlich in erfindungsgemäßer Weise solche eingesetzt werden, die ein bewegliches Halogenatom oder Halogenidion enthalten und die nicht unter die Bestandteile der Formel (III) fallen. Für den Fall, daß R³ Wasserstoff bedeutet, sind dies Chlorwasserstoff, Bromwasserstoff, Iodwasserstoff und ihre Salze mit Stickstoff- oder Phosphorbasen, wie beispielsweise Ammoniumhalogenide, Phosphoniumhalogenide, Pyridiniumhalogenide. Für den Fall, daß R³ Chlor, Brom oder Iod, Cl₃ oder Br₃ bedeutet, sind die Halogenverbindungen die Halogene Cl₂, Br₂ oder J₂ sowie die Interhalogenverbindungen, wie Bromchlorid, Iodtrichlorid, Iodtribromid. Für den Fall, daß R³ den Rest eines anorganischen oder organischen Säurehalogenids einschließlich Phenacyl darstellt, umfaßt die Halogenverbindung (IV) Verbindungen, wie Thionylhalogenide, Sulfurylhalogenide, Phosphortrihalogenide, Phosphorpentahalogenide, Phosphoroxitrihalogenide als wichtige Vertreter anorganischer Säurehalogenide und Acetylhalogenide, Propionylhalogenide, Butyrylhalogenide, Benzoylhalogenide und durch Methyl, Chlor, Brom oder Hydroxy substituierte Benzoylhalogenide als wichtige Vertreter organischer Säurehalogenide und z.B. 2-Haloethanol als wichtiges Beispiel eines β-Halogenalkanols.

Weitere Halohydrine sind beispielsweise:
2-Chlorethanol, 2-Bromethanol, 2-Iodethanol, 1-Chlor-2-propanol, 2-Chlor-1-propanol, 1-Brom-2-propanol, 2-Brom-1-propanol, 1-Iod-2-propanol, 2-Iod-1-propanol, 3-Chlor-2-butanol, 3-Brom-2-butanol, 3-Iod-2-butanol, 1-Brom-2-butanol, 3-Iod-2-butanol, 1-Brom-2-butanol, 2-Brom-1-butanol, 2-Chlor-2-phenylethanol, 2-Brom-2-phenyl-ethanol, 2-Iod-2-phenylethanol, 2-Chlor-1-phenylethanol, 2-Brom-1-phenylethanol, 2-Iod-1-phenylethanol, 1,3-Dichlor-2-propanol, 2,3-Dichlorpropanol, 1-Brom-3-chlor-2-propanol, 2-Brom-3-chlorpropanol, 1-Iod-3-chlor-2-propanol, 2-Iod-3-chlorpropanol, usw.

Für den Fall, daß R³ C₄-C₈-tert.-Alkyl bedeutet, stellt die Halogenverbindung (IV) tert.-Butylhalogenid oder tert.-Amylhalogenid dar. R³ kann ferner (Meth)Allyl bedeuten.

In bevorzugter Weise werden zur Aktivierung Halogenverbindungen der Formel

R¹³-Z (V)

eingesetzt, in der
- R¹³: Wasserstoff, C₄-C₆-tert.-Alkyl, -CHR¹¹-CHR¹²-OH bedeutet, worin R¹¹ und R¹² unabhängig voneinander für H, CH₃, C₂H₅ oder Phenyl stehen, und
- Z: den oben angegebenen Bedeutungsumfang hat.

Besonders bevorzugte Aktivatoren sind Halogenverbindungen der Formel

R²³ - Br (VI),

in der
- R²³: Wasserstoff oder -CHR¹¹-CHR¹²-OH bedeutet, worin R¹¹ und R¹² die obige Bedeutung annehmen.

In den Katalysatoren der Formel (III) liegt das Verhältnis der gebrochenen oder ganzen Zahlen a und b bevorzugt m einem Bereich für a:b = 20:1-1:5, besonders bevorzugt 10:1-1:2, ganz besonders bevorzugt 3:1-1:1.

Eine bevorzugte Form des Katalysators ist auch eine solche, bei der man Alkalihalogenid und Zinkhalogenid im Molverhältnis 2:1 oder separat hergestellte Komplexe aus Alkalihalogenid und Zinkhalogenid einsetzt.

Das einzusetzende Kohlendioxid kann mit inerten Gasen, wie mit Stickstoff, Wasserstoff, Kohlenmonoxid, niederen Kohlenwasserstoffen verunreinigt sein oder aus natürlichen Quellen oder industriellen Abgasen stammen.

Das einzusetzende Alkylenoxid kann entweder technisch übliches reines Alkylenoxid sein oder ein Alkylenoxid enthaltendes Rohprodukt sein, wie es beispielsweise bei der Sauerstoffoxidation von Alkenen an metallhaltigen Katalysatoren, wie beispielsweise Silberkatalysatoren, anfällt.

Der Reaktionsdruck im erfindungsgemäßen Verfahren liegt im allgemeinen bei einem absoluten Reaktionsdruck von unter 30 bar, bevorzugt von unter 20 bar, besonders bevorzugt von unter 15 bar.

Die Reaktionstemperatur liegt bei 40-250°C, bevorzugt 50-200°C, besonders bevorzugt bei 70-170°C.

Das Molverhältnis von Alkylenoxid und Kohlendioxid liegt im Prinzip bei 1:1, ein Überschuß an Kohlendioxid ist jedoch möglich. Das Molverhältnis Alkylenoxid zu Kohlendioxid liegt demnach erfindungsgemäß im Bereich von 1:1-10, bevorzugt bei 1:1-3 und besonders bevorzugt bei 1:1-1,5.

Die Menge des Katalysators der Formel (III) liegt, bezogen auf das im Reaktionssystem befindliche Alkylenoxid, bei 0,001-10 Gew.-%, bevorzugt bei 0,005-5 Gew.-%, besonders bevorzugt bei 0,01-2 Gew.-%, ganz besonders bevorzugt bei 0,02-1 Gew.-%.

Die Umsetzung wird in dem jeweils herzustellenden Alkylencarbonat als Reaktionsmedium durchgeführt, also in Ethylencarbonat, wenn Ethylenoxid mit Kohlendioxid umgesetzt werden soll, bzw. in Propylencarbonat, wenn Propylenoxid mit Kohlendioxid umgesetzt werden soll. Es ist selbstverständlich auch möglich, beispielsweise Ethylenoxid mit Kohlendioxid in Propylencarbonat zur Reaktion zu bringen; diese Variante ist jedoch nicht bevorzugt.

Es ist weiterhin möglich, das Reaktionsmedium durch unter den Reaktionsbedingungen inerte Stoffe zu verdünnen. Dies sind beispielsweise die dem Fachmann bekannten Lösungsmittel, wie beispielsweise aliphatische Kohlenwasserstoffe (Decan, Octadecan, Decalin und andere), aromatische Kohlenwasserstoffe (Benzol, Toluol, Xylol, Mesitylen, Tetrahydronaphthalin und andere), cyclische Amide (N-Methylpyrrolidon, N-Methyl-caprolactam und andere), Harnstoffe (N,N'-Dimethyl-imidazolidin-2-on); des weiteren können inerte Gase, wie Stickstoff, Wasserstoff, Argon und andere mitverwendet werden. In bevorzugter Weise ist die Fahrweise ohne Zusatz solcher inerter Stoffe.

Falls eine Verdünnung des Reaktionsmediums mit einem dieser Lösungsmittel gewählt wird, ist es weiterhin möglich, zum Start der Umsetzung dieses Lösungsmittel und den Katalysator ohne Zusatz des herzustellenden Alkylencarbonats als Reaktionsmedium vorzulegen. Im Verlaufe der Umsetzung entsteht dann durch die Bildung von Alkylencarbonat ein Reaktionsmedium, das ein Gemisch aus diesem Lösungsmittel und dem Alkylencarbonat darstellt.

Das erfindungsgemäße Verfahren ist grundsätzlich ohne aktivierende Halogenverbindung durchführbar. Es ist jedoch bevorzugt, daß der bei der Aufarbeitung des Reaktionsgemisches anfallende Katalysator vor seinem Wiedereinsatz durch Behandlung mit einer Halogenverbindung (IV) aktiviert wird. Eine solche Aktivierung kann auch bereits vor dem ersten Einsatz des Katalysators erfolgen. Dies gilt besonders für die Aktivierung mit Halogenverbindung der Formel

Z - CHR¹ - CHR² - OH,

worin Z, R¹ und R² die obige Bedeutung haben, und besonders für Halohydrinverbindungen, die unter die Formel (VI) fallen.

Die Halohydrine können als solche dem Reaktionsgemisch zugesetzt werden. Sie können aber auch im Reaktionsgemisch durch Umsetzung des erfindungsgemäß eingesetzten Alkylenoxids der Formel (I) mit Halogenwasserstoff H-Z oder mit Halogenwasserstoff abspaltenden Stoffen oder Stoffgemischen erzeugt werden. Es ist bevorzugt, die Halohydrine als solche zuzusetzen oder sie durch Einleiten von Halogenwasserstoff in dem Reaktionsgemisch zu erzeugen.

Die Menge des in dem Reaktionsgemisch befindlichen Halohydrins ist im Prinzip beliebig, auch ein sehr hoher Überschuß an Halohydrin stört die erfindungsgemäße Umsetzung nicht. Im allgemeinen liegt die in dem Halohydrin enthaltende Halogenmenge, bezogen auf die Halogenmenge in der eingesetzten Verbindung der Formel (III), in einem Molverhältnis Z zu X+Y von 0,001 zu 10, bevorzugt bei 0,01 zu 5, besonders bevorzugt bei 0,05 bis 1, ganz besonders bevorzugt bei 0,1 bis 0,5.

Die Menge der weiteren erfindungsgemäß zur Aktivierung einzusetzenden Halogenverbindung (IV) beträgt, bezogen auf die Summe der im Reaktionsgemisch vorliegenden Verbindungen der Formel (III), das 0,0001- bis 100fache, bevorzugt das 0,0002- bis 75fache, besonders bevorzugt das 0,0005- bis 50fache, ganz besonders bevorzugt das 0,001-bis 25fache dieser Menge.

Etwa in das Reaktionsmedium eingebrachtes Wasser stört die erfindungsgemäße Umsetzung nicht; es reagiert mit dem Alkylencarbonat unter Bildung von Kohlendioxid und dem zugehörigen Glykolderivat. Es ist jedoch bevorzugt, alle Einsatzstoffe mit demjenigen niedrigen Wassergehalt einzusetzen, mit dem sie üblicherweise in der chemischen Technik vorliegen.

Es ist erfindungsgemäß bevorzugt, daß bei dem Reaktionsgemisch die Verbindungen der Formeln (I), (IV), (V) und (VI) die gleichen Reste R¹ und die gleichen Reste R² tragen, d.h. wenn z.B. der Einsatzstoff der Formel (I) Ethylenoxid bedeutet, daß beispielsweise 2-Bromethanol eingesetzt wird, oder wenn beispielsweise der Stoff der Formel (I) Propylenoxid bedeutet, daß (IV) ein 2-Halo-1-propanol oder ein 1-Halo-2-propanol bedeutet.

Es ist jedoch erfindungsgemäß ebenfalls möglich, daß die Einsatzstoffe der Formel (I) und die der Formeln (IV), (V) und (VI) untereinander verschiedene Reste R¹ und untereinander verschiedene Reste R² tragen.

Das erfindungsgemäße Verfahren kann in verschiedenen Ausführungsvarianten ausgeübt werden:
Eine Möglichkeit besteht in der diskontinuierlichen Durchführung in üblichen Rührgefäßen oder Blasensäulen. Dabei können solange Alkylenoxid und Kohlendioxid zugegeben werden, bis das Gefäß gefüllt ist. Dann wird das gebildete Alkylencarbonat abdestilliert. In den zurückbleibenden Sumpf gibt man erneut Alkylenoxid und Kohlendioxid. Bei nachlassender Katalysatoraktivität gibt man entweder vor, während oder nach dem Abdestillieren des gebildeten Alkylencarbonats die zur Aktivierung vorgesehene Halogenverbindung (IV) zu. Es ist gleichermaßen möglich, die Aktivierung des Katalysators (III) mit der Halogenverbindung (IV) auch nur in einem Teil des Reaktionsmediums bzw. in einem Teil des nach dem Abdestillieren des Alkylencarbonats erhaltenen Sumpfes vorzunehmen. Alle Sümpfe werden danach vereinigt und wieder dem Reaktionsgefäß zum erneuten Einsatz zugeführt.

Eine weitere Reaktionsvariante betrifft die bevorzugte kontinuierliche Reaktionsführung. Als Reaktionsgefäß kommen dabei übliche Rührkessel, Blasensäulen oder Kessel- oder Blasensäulenkaskaden in Frage, die wiederum in verschiedenen Schaltungen, beispielsweise in Serie und/oder parallel zueinander angelegt werden könnnen. Kohlendioxid und Alkylenoxid werden kontinuierlich eindosiert, und das Alkylencarbonat wird zusammen mit dem darin enthaltenen Katalysator abgeführt. Aus diesem abgeführten Strom wird, entsprechend der neu gebildeten Menge, Alkylencarbonat vom Katalysatorsystem abgetrennt, beispielsweise durch Membranen oder durch Destillation; der katalysatorhaltige Rest wird sodann ganz oder teilweise dem Reaktionsgefäß zugeführt oder ganz oder teilweise zur Aktivierung mit der vorgesehenen Halogenverbindung (IV) versetzt.

Bei diskontinuierlicher oder kontinuierlicher Ausführung wird beispielsweise zunächst zu Beginn der Reaktion eine kleine Menge des herzustellenden Alkylencarbonats zusammen mit dem Katalysator der Formel (III) ohne Zugabe von Halogenverbindung, beispielsweise von Halohydrin oder Halohydrin erzeugenden Stoffen vorgelegt. Dann wird die Zugabe von Alkylenoxid und Kohlendioxid begonnen. Nach dem Ende der diskontinuierlichen Zugabe oder bei kontinuierlich abgeführtem Strom wird von dem Reaktionsgemisch eine Leichtsiederfraktion abgenommen, die während der Umsetzung durch Nebenreaktion des Katalysators mit Alkylencarbonat und vorhandenen oder in dem zudosierten Alkylenoxid oder Kohlendioxid enthaltenen, protischen Verbindungen gebildetes Halohydrin enthält. Diese Leichtsiederfraktion wird gemeinsam mit dem Sumpf, der den Katalysator (III) enthält, in die diskontinuierliche oder kontinuierliche Umsetzung zurückgeführt.

Es ist ebenfalls erfindungsgemäß bevorzugt, bei diskontinuierlicher oder kontinuierlicher Fahrweise und nach Zusatz eines Halohydrins zu dem Startgemisch aus einer kleinen Menge Alkylencarbonat und dem Katalysator der Formel (III) und nach Durchführung der Umsetzung von Alkylenoxid und Kohlendioxid bei der Abdestillation des neu gebildeten Alkylencarbonats eine Leichtersiederfraktion zu entnehmen, die das ursprünglich eingesetzte und evtl. neu gebildetes Halohydrin enthält, und diese Leichtsiederfraktion zurückzuführen.

Ebenfalls bevorzugt ist eine kontinuierliche Zudosierung von Halohydrin in eine kontinuierliche Umsetzung von Alkylenoxid mit CO₂, wobei das Halohydrin sowohl vor der Einspeisung als auch nach der Einspeisung im Reaktionssystem aus Halogenwasserstoff und Alkylenoxid bzw. Alkylencarbonat in situ erzeugt werden kann.

Das erfindungsgemäße Verfahren ist in verschiedener Hinsicht ausgesprochen überraschend. In der genannten allgemeinen chemischen Literatur werden ausdrücklich iodidhaltige Katalysatoren als essentiell für gute katalytische Wirkung genannt. So ist völlig unerwartet, daß ein auch Brom enthaltendes Gemisch aus einfachen Alkalihalogeniden und Zinkhalogenid eine ausgezeichnete und eine die übrigen bereits beschriebenen Systeme übertreffende katalytische Aktivität zeigt, so daß selbst bei Normaldruck die Umsetzung in kurzer Zeit abläuft. Weiterhin überraschend ist, daß gerade dieses Brom enthaltende Gemisch mehrfach rückführbar ist und dann nach etwaigem Aktivitätsverlust durch Zugabe von beispielsweise Bromverbindungen, die nicht zu dem Katalysatorsystem selbst gehören, aktivierbar ist. Solche Reaktivierungen von desaktivierten Katalysatoren zur Umsetzung von Alkylenoxiden mit Kohlendioxid sind bisher nicht beschrieben worden.

Die folgenden Beispiele verdeutlichen das erfindungsgemäße Verfahren, ohne es jedoch auf diese einzuschränken.

### Beispiel 1

In ein senkrecht stehendes Rohr von 100 cm Länge und 3 cm Durchmesser, das mit einem Ölheizmantel und am unteren Ende mit einer Gaseinleitefritte versehen ist, wurden 700 g Ethylencarbonat, das 1,30 g Zinkbromid und 2,50 g Kaliumiodid als Katalysatoren gelöst enthielt, eingefüllt und auf 120°C vorgeheizt. Bei dieser Temperatur wurden in 4 h 74 g Ethylenoxid und 82 g Kohlendioxid als Gasgemisch gleichmäßig durch die Bodenfritte eingeleitet. Das Gasgemisch wurde weitgehend absorbiert. Nach dem Ende der Gaseinleitung wurde das Gemisch in einen Kolben abgelassen und ausgewogen. Die so auswiegbare Gewichtszunahme betrug 146 g. Dies entspricht unter Berücksichtigung des Umfüllverlustes einer fast quantitativen Umwandlung des Ethylenoxids. Die gaschromatographische Analyse des Endproduktes ergab, daß keine Nebenprodukte gebildet wurden. Anschließend wurden bei 18-22 mbar 160 g an Ethylencarbonat von diesem Ansatz abdestilliert. Der zurückbleibende Sumpf wurde wieder in die Blasensäule eingefüllt und in 4 h gleichmäßig mit 75 g Ethylenoxid und 83 g CO₂ begast. Die Gewichtszunahme war 148 g.

### Beispiel 2

Das Verfahren des Beispiels 2 wurde wiederholt, nur daß hier 2,14 g Zinkbromid und 1,97 g Natriumbromid als Katalysatoren eingesetzt wurden. Die in 4 h bei 120°C eingeleiteten Gasmengen waren 72 g Ethylenoxid und 82 g Kohlendioxid. Die nach dem Ablassen auswiegbare Gewichtszunahme betrug 142 g. Wie in Beispiel 1 wurden 173 g Ethylencarbonat abdestilliert und der Sumpf rückgeführt. Die erneut eingeleitete Menge betrug 75 g Ethylenoxid und 85 g CO₂. Die erneute Gewichtszunahme betrug 146 g.

### Vergleichsbeispiel 3

Das Verfahrens des Beispiels 1 wurde wiederholt, nur daß man hier 1,3 g Zinkchlorid und 5,0 g Tetrabutylammoniumiodid einsetzte. In 4 h bei 120°C wurden 73 g Ethylenoxid und 77 g CO₂ eingeleitet. Die Zunahme betrug 122 g. Nach dem Abdestillieren von 155 g Ethylencarbonat bei 20-22 mbar wurde der Sumpf erneut bei 120°C mit 70 g Ethylenoxid und 77 g CO₂ begast. Die Zunahme betrug nur noch 33 g.

### Beispiel 4

Das Verfahren des Beispiels 2 wurde wiederholt, nur daß hier die halbe Menge an beiden Katalysatoren, d.h. 1,07 g Zinkbromid und 0,98 g Natriumbromid, eingesetzt wurde. Gleichzeitig wurde die in 4 h eingeleitete Gasmenge so stark erhöht, daß aufgrund der kurzen (ca. 70-80 cm) überstehenden Flüssigkeitssäule keine vollständige Absorption des Ethylenoxids mehr möglich war. Die eingeleiteten Gasmengen sind in der Tabelle zum Beispiel 4 angegeben. Nach der Bestimmung der Auswaage wurde in einem Vakuum von 20-25 mbar die gebildete Menge (ca. 150-250 g) an Ethylencarbonat abdestilliert und der zurückbleibende Sumpf zur Rückführung der Katalysatoren erneut in das senkrecht stehende Rohr eingefüllt. Nachdem die Katalysatoren auf diese Art fünfmal rückgeführt worden waren, wurde nach der Destillation bei 20-25 mbar und vor dem Wiedereinsatz in das Reaktionsrohr in den ca. 120°C warmen Destillationssumpf 4 g HBr in 15 min gleichmäßig zur Reaktivierung eingeblasen. Danach wurde der aktivierte Sumpf erneut in das Reaktionsrohr vorgelegt und weitere dreimal rückgeführt. Danach wurden wiederum 4 g HBr in den Sumpf nach der Destillation und vor dem erneuten Einfüllen eingeleitet. Anschließend wurde der Sumpf zur neunten Rückführung, d.h. insgesamten zehnten Verwendung, in die Blasensäule eingefüllt. Die Ergebnisse befinden sich in der Tabelle zum Beispiel 4.

| Tabelle zum Beispiel 4 | | | | |
|---|---|---|---|---|
| | Eingeleitete Gasmengen | | Gewichtszunahme/g | Ausbeute EC bez. auf EOX in % |
| | EOX/g | CO₂/g | | |
| Frischansatz | 124 | 140 | 206 | 83,1 |
| 1. Rf | 124 | 137 | 199 | 80,2 |
| 2. Rf | 126 | 144 | 197 | 78,2 |
| 3. Rf | 125 | 145 | 189 | 75,6 |
| 4. Rf | 120 | 146 | 165 | 68,8 |
| 5. Rf | 124 | 140 | 132 | 53,2 |

| Reaktivierung durch HBr | | | | |
|---|---|---|---|---|
| 6. Rf | 120 | 143 | 175 | 72,9 |
| 7. Rf | 122 | 144 | 193 | 79,1 |
| 8. Rf | 125 | 140 | 189 | 75,6 |

| Reaktivierung durch HBr | | | | |
|---|---|---|---|---|
| 9. Rf | 125 | 141 | 201 | 80,4 |
| Reaktionstemperatur: 120°C 1,07 g ZnBr₂/0,98 g NaBr in 700 g Ethylencarbonat Einleitezeit der Gase 4 h Rf: Rückführung EOX: Ethylenoxid EC: Ethylencarbonat | | | | |

### Beispiel 5

Das Verfahren des Beispiels 4 wurde wiederholt, nur daß hier 1,07 g Zinkbromid und statt 0,98 g Natriumbromid 0,70 g Natriumbromid eingesetzt wurden. Das nach der Auswaage durchgeführte Abdestillieren des gebildeten Anteils an Ethylencarbonat erfolgte in gleicher Weise wie bei Beispiel 4. Anstelle von HBr wurden nach der 4. Rückführung 4 g 2-Bromethanol in den ca. 100°C warmen Sumpf zugegeben. Die Ergebnisse finden sich in der Tabelle zum Beispiel 5.

| Tabelle zum Beispiel 5 | | | | |
|---|---|---|---|---|
| | Eingeleitete Gasmengen | | Gewichtszunahme/g | Ausbeute EC bez. auf EOX in % |
| | EOX/g | CO₂/g | | |
| Frischansatz | 122 | 138 | 175 | 75,8 |
| 1. Rf | 120 | 140 | 176 | 73,3 |
| 2. Rf | 122 | 140 | 159 | 65,2 |
| 4. Rf | 124 | 145 | 90 | 36,3 |

| Reaktivierung durch 2-Bromethanol | | | | |
|---|---|---|---|---|
| 5. Rf | 120 | 140 | 134 | 55,8 |
| 6. Rf | 121 | 138 | 160 | 66,1 |
| 7. Rf | 119 | 138 | 158 | 66,4 |
| Reaktionstemperatur: 120°C 1,07 g ZnBr₂/0,98 g NaBr in 700 g Ethylencarbonat Einleitezeit der Gase 4 h Rf: Rückführung EOX: Ethylenoxid EC: Ethylencarbonat | | | | |

### Beispiel 6

Das Verfahren des Beispiels 4 wurde wiederholt, nur daß statt Zinkbromid hier 0,65 g Zinkchlorid und 0,98 g Natriumbromid eingesetzt wurden. Nach der 1. Rückführung wurde durch Einleiten von 2 g HBr in den ca. 120°C heißen Sumpf reaktiviert. Die Ergebnisse finden sich in der Tabelle zum Beispiel 6.

| Tabelle zum Beispiel 6 | | | | |
|---|---|---|---|---|
| | Eingeleitete Gasmengen | | Gewichtszunahme/g | Ausbeute EC bez. auf EOX in % |
| | EOX/g | CO₂/g | | |
| Frischansatz | 118 | 129 | 126 | 53,4 |
| 1. Rf | 120 | 122 | 64 | 26,7 |

| Reaktivierung durch HBr | | | | |
|---|---|---|---|---|
| 2. Rf | 115 | 133 | 116 | 50,4 |
| 3. Rf | 118 | 128 | 132 | 55,9 |
| Reaktionstemperatur: 120°C 0,65 g ZnCl₂/0,98 g NaBr in 700 g Ethylencarbonat Einleitezeit der Gase 4 h Rf: Rückführung EOX: Ethylenoxid EC: Ethylencarbonat | | | | |

### Vergleichsbeispiel 7

Das Verfahren des Beispiels 4 wurde wiederholt, nur daß statt der dortigen Katalysatoren 0,67 g Zinkchlorid und 2,55 g Tetrabutylammoniumiodid eingesetzt wurden. Nach der ersten Rückführung wurden 3 g HBr in den ca. 110-120°C heißen Sumpf eingeleitet. Das Ergebnis findet sich in der Tabelle zum Vergleichsbeispiel 7.

| Tabelle zum Vergleichsbeispiel 7 | | | | |
|---|---|---|---|---|
| | Eingeleitete Gasmengen | | Gewichtszunahme/g | Ausbeute EC bez. auf EOX in % |
| | EOX/g | CO₂/g | | |
| Frischansatz | 118 | 129 | 68 | 28,8 |
| 1. Rf | 120 | 133 | 8 | 3,3 |

| Reaktivierung mit HBr | | | | |
|---|---|---|---|---|
| 2. Rf | 115 | 125 | 1 | 0,4 |
| Reaktionstemperatur: 120°C 0,67 g ZnCl₂/2,55 g Tetrabutylammoniumiodid in 700 g Ethylencarbonat Einleitezeit der Gase 4 h Rf: Rückführung EOX:Ethylenoxid EC: Ethylencarbonat | | | | |

### Beispiel 8

Das Verfahren des Beispiels 4 wurde wiederholt, nur daß statt der dortigen Katalysatorn 1,07 g Zinkbromid und 1,12 g Kaliumiodid eingesetzt wurden. Nach der zweiten Rückführung wurden in die ausgewogene zum Abdestillieren des gebildeten Ethylencarbonats eingesetzte Reaktionsmischung 2,5 g 2-Iodethanol gegeben, dann bei 20 mbar der gebildete Teil abdestilliert (ca. 100-200 g) und der zurückbleibende Sumpf wie üblich in die Blasensäule zur Rückführung eingefüllt. Das Ergebnis findet man in der Tabelle zum Beispiel 8.

| Tabelle zum Beispiel 8 | | | | |
|---|---|---|---|---|
| | Eingeleitete Gasmengen | | Gewichtszunahme/g | Ausbeute EC bez. auf EOX in % |
| | EOX/g | CO₂/g | | |
| Frischansatz | 120 | 129 | 158 | 65,8 |
| 1. Rf | 115 | 133 | 145 | 63,0 |

| Reaktivierung durch HBr | | | | |
|---|---|---|---|---|
| 3. Rf | 117 | 130 | 137 | 58,6 |
| 4. Rf | 112 | 119 | 139 | 62,1 |
| Reaktionstemperatur: 120°C 1,07 g Zinkbromid/1,12 g Kaliumiodid in 700 g Ethylencarbonat Einleitezeit der Gase 4 h Rf: Rückführung EOX: Ethylenoxid EC: Ethylencarbonat | | | | |

### Beispiel 9

Das Verfahren des Beispiels 4 wurde wiederholt, nur wurde hier nach jedem Abdestillieren des gebildeten Ethylencarbonats ca. 1-2 g HBr in 2-3 min gasförmig in den 100-120°C heißen Sumpf eingeleitet und der zurückbleibende Sumpf in die Blasensäule rückgeführt. Das Ergebnis findet sich in der Tabelle zum Beispiel 9.

| Tabelle zum Beispiel 9 | | | | |
|---|---|---|---|---|
| | Eingeleitete Gasmengen | | Gewichtszunahme/g | Ausbeute EC bez. auf EOX in % |
| | EOX/g | CO₂/g | | |
| Frischansatz | 124 | 140 | 204 | 82,3 |

| Reaktivierung mit HBr | | | | |
|---|---|---|---|---|
| 1. Rf | 126 | 138 | 203 | 80,6 |

| Reaktivierung mit HBr | | | | |
|---|---|---|---|---|
| 2. Rf | 130 | 139 | 203 | 78,1 |

| Reaktivierung mit HBr | | | | |
|---|---|---|---|---|
| 3. Rf | 122 | 144 | 198 | 81,2 |

| Reaktivierung mit HBr | | | | |
|---|---|---|---|---|
| 4. Rf | 125 | 141 | 196 | 78,4 |

| Reaktivierung mit HBr | | | | |
|---|---|---|---|---|
| 5. Rf | 127 | 142 | 202 | 79,5 |
| Reaktionstemperatur: 120°C 1,07 g ZnBr₂/0,98 g NaBr in 700 g Ethylencarbonat Einleitezeit der Gase 4 h Rf: Rückführung EOX: Ethylenoxid EC: Ethylencarbonat | | | | |

### Beispiel 10

Das Verfahren des Beispiels 1 wurde wiederholt, nur daß man statt der dortigen Katalysatoren 2,05 g an Na₂[ZnBr₄] einsetzte. Bei 120°C wurden 68 g EOX und 80 g CO₂ in 4 h eingeleitet. Die Zunahme betrug 133 g. Nach dem Abdestillieren von 149 g Ethylencarbonat bei 20-25 mbar wurde der Sumpf erneut eingesetzt. Man leitete bei 120°C 75 g EOX und 82 g CO₂ ein. Die Gewichtszunahme betrug 147 g.

### Beispiel 11

In ein senkrecht stehendes Rohr von 110 cm Länge und 3 cm Durchmesser, das mit einem Ölheizmantel und am unteren Ende mit einer Gaseinleitefritte versehen ist, wurden 700 g Ethylencarbonat, 1,96 g Natriumbromid, 2,16 g Zinkbromid und 0,93 g 2-Bromethanol eingefüllt und auf 120°C vorgeheizt. Bei dieser Temperatur wurden in 4 Stunden 78 g Ethylenoxid und 86 g Kohlendioxid als Gasgemisch gleichmäßig durch die Bodenfritte eingeleitet. Das Gasgemisch wurde weitgehend absorbiert. Nach dem Ende der Gaseinleitung wurde das Gemisch in einen Kolben abgelassen und ausgewogen. Die so auswiegbare Gewichtszunahme betrug 153,5 g. Dies entsprach unter Berücksichtigung des Umfüllverlustes einer quantitativen Umsetzung des Ethylenoxids.

Das Reaktionsprodukt wurde in eine Vakuumdestillationsapparatur umgefüllt, und bei 20 bis 22 mbar wurde über eine verspiegelte 50 cm-Vigreux-Kolonne bis zu einer Kopftemperatur von 122°C ein Vorlauf (15,3 g) abgenommen. Dieser Vorlauf enthielt das 2-Bromethanol.

### Beispiel 12

Das Verfahren des Beispiels 11 wurde wiederholt, nur daß hier 0,46 g 2-Bromethanol, 1,07 g Zinkbromid und 0,98 g Natriumbromid eingesetzt wurden. Gleichzeitig wurde die in 4 Stunden eingeleitete Gasmenge so stark erhöht, daß aufgrund der kurzen (ca 70 bis 80 cm) überstehenden Flüssigkeitssäule keine vollständige Absorption des Ethylenoxids mehr möglich war. Die eingeleiteten Gasmengen befinden sich in der Tabelle zum Beispiel 12. Nach der Bestimmung der Auswaage wurde bei einem Vakuum von ca. 40 mbar die gebildete Menge (ca. 200 g) an Ethylenglykolcarbonat abdestilliert und der zurückbleibende Sumpf nach Zugabe von 0,45 bis 0,47 g 2-Bromethanol erneut zur Rückführung der Katalysatoren in das senkrecht stehende Rohr eingefüllt. Das Ergebnis findet man in der Tabelle zum Beispiel 12.

| Tabelle zum Beispiel 12 | | | | |
|---|---|---|---|---|
| | Eingeleitete Gasmengen | | Gewichtszunahme/g | Ausbeute EC, bez. auf EOX, in % |
| | EOX/g | CO₂/g | | |
| Frischansatz | 122 | 140 | 198 | 81,1 |
| 1. Rf | 124 | 138 | 196 | 79,0 |
| 2. Rf | 125 | 140 | 196 | 78,4 |
| 3. Rf | 122 | 140 | 193 | 79,1 |
| 4. Rf | 125 | 144 | 197 | 78,8 |
| 5. Rf | 123 | 141 | 194 | 78,9 |
| 6. Rf | 120 | 142 | 191 | 79,6 |
| 7. Rf | 118 | 140 | 186 | 78,8 |
| 8. Rf | 122 | 139 | 193 | 79,1 |
| 9. Rf | 121 | 140 | 194 | 80,1 |
| Reaktionstemperatur: 120°C Rf: Rückführung EOX = Ethylenoxid EC: Ethylencarbonat | | | | |

### Beispiel 13

Das Verfahren des Beispiels 12 wurde wiederholt, nur daß vor der Rückführung des zurückbleibenden Sumpfes kein 2-Bromethanol zugegeben wurde. Das Ergebnis findet man in der Tabelle zum Beispiel 13.

| Tabelle zum Beispiel 13 | | | | |
|---|---|---|---|---|
| | Eingeleitete Gasmengen | | Gewichtszunahme/g | Ausbeute EC, bez. auf EOX, in % |
| | EOX/g | CO₂/g | | |
| Frischansatz | 123 | 139 | 201 | 81,7 |
| 1. Rf | 124 | 140 | 193 | 77,8 |
| 2. Rf | 124 | 138 | 115 | 46,4 |
| 3. Rf | 120 | 144 | 70 | 28,2 |
| 4. Rf | 122 | 151 | 57 | 23,4 |
| Reaktionstemperatur: 120°C | | | | |

### Beispiel 14

Das Verfahren des Beispiels 12 wurde wiederholt, nur daß statt einer einfachen Vakuumdestillation nun über eine 50 cm-Vigreux-Kolonne bei 40 mbar bis 136°C Kopftemperatur ein Leichtsiedervorlauf entnommen wurde (ca. 20 g). Anschließend wurde die restliche neugebildete Menge Ethylencarbonat bei 40 mbar ebenfalls über diese Kolonne destilliert. Der zurückbleibende Sumpf und der Leichtsiedervorlauf wurden zur Rückführung des Halohydrins und der Katalysatoren in das senkrecht stehende Rohr eingefüllt. Das Ergebnis findet man in der Tabelle zum Beispiel 14.

| Tabelle zum Beispiel 14 | | | | |
|---|---|---|---|---|
| | Eingeleitete Gasmengen | | Gewichtszunahme/g | Ausbeute EC, bez. auf EOX, in % |
| | EOX/g | CO₂/g | | |
| Frischansatz | 120 | 138 | 193 | 80,4 |
| 1. Rf | 118 | 140 | 188 | 79,6 |
| 2. Rf | 122 | 142 | 192 | 78,7 |
| 3. Rf | 120 | 138 | 188 | 78,3 |
| 4. Rf | 120 | 139 | 186 | 77,5 |
| 5. Rf | 119 | 142 | 185 | 77,7 |
| Reaktionstemperatur: 120°C Zusatz von 2-Bromethanol nur in den Frischansatz Rückführung von Leichtsiederfraktion und zurückbleibendem Sumpf | | | | |

### Beispiel 15

Das Verfahren des Beispiels 12 wurde wiederholt, nur daß hier 0,23 g 2-Bromethanol, 0,41 g 2-Iodethanol, 1,07 g Zinkbromid und 1,58 g Kaliumiodid eingesetzt wurden.

Nach dem Abdestillieren des gebildeten Ethylencarbonats wurde vor der Rückfuhrung des zurückbleibenden Sumpfes jeweils 0,22 bis 0,25 g 2-Bromethanol und 0,40 bis 0,43 g 2-Iodethanol zugegeben. Das Ergebnis findet man in der Tabelle zum Beispiel 15.

| Tabelle zum Beispiel 15 | | | | |
|---|---|---|---|---|
| | Eingeleitete Gasmengen | | Gewichtszunahme/g | Ausbeute EC, bez. auf EOX, in % |
| | EOX/g | CO₂/g | | |
| Frischansatz | 122 | 140 | 200 | 82,0 |
| 1. Rf | 116 | 144 | 190 | 81,9 |
| 2. Rf | 122 | 142 | 193 | 79,1 |
| 3. Rf | 121 | 141 | 192 | 79,3 |
| 4. Rf | 123 | 145 | 194 | 78,9 |
| Reaktionstemperatur: 120°C | | | | |

### Beispiel 16

Das Verfahren des Beispiels 12 wurde wiederholt, nur daß hier 0,45 g 2-Bromethanol, 0,65 g Zinkchlorid und 0,99 g Natriumbromid eingesetzt wurden. Nach dem Abdestillieren des gebildeten Ethylencarbonats wurde vor der Rückführung des zurückbleibenden Sumpfes jeweils 0,43 bis 0,46 g 2-Bromethanol zugegeben. Das Ergebnis findet man in der Tabelle zum Beispiel 16.

| Tabelle zum Beispiel 16 | | | | |
|---|---|---|---|---|
| | Eingeleitete Gasmengen | | Gewichtszunahme/g | Ausbeute EC, bez. auf EOX, in % |
| | EOX/g | CO₂/g | | |
| Frischansatz | 123 | 143 | 192 | 78,0 |
| 1. Rf | 125 | 146 | 198 | 79,2 |
| 2. Rf | 122 | 144 | 196 | 80,3 |
| 3. Rf | 119 | 142 | 191 | 80,2 |
| 4. Rf | 120 | 144 | 191 | 79,5 |
| Reaktionstemperatur: 120°C | | | | |

## Patentansprüche

1. Verfahren zur Herstellung von Alkylencarbonaten der Formel 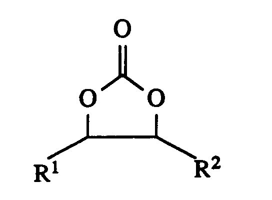 durch Umsetzung von Alkylenoxiden der Formel 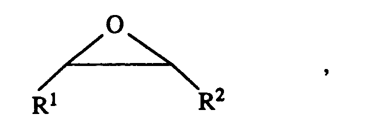 wobei in den Formeln
R¹ und R² unabhängig voneinander Wasserstoff, substituiertes oder nicht substituiertes C₁-C₄-Alkyl, substituiertes oder nicht substituiertes C₂-C₄-Alkenyl oder substituiertes oder nicht substituiertes C₆-C₁₂-Aryl bedeuten und
R¹ und R² gemeinsam mit den beiden Dreiring-C-Atomen einen gesättigten carbocyclischen Ring mit 5-8 Ringgliedern bedeuten können,
mit Kohlendioxid in Gegenwart von Katalysatoren, dadurch gekennzeichnet, daß man aktivierbare Katalysatoren der Formel
a[MX]/b[ZnY₂]
einsetzt, worin
M ein Alkalimetall, bevorzugt Li, Na oder K, besonders bevorzugt Na oder K, bedeutet,
X und Y unabhängig voneinander Chlor, Brom oder Iod bedeuten und
a und b gebrochene oder ganze Zahlen in einem Bereich von 0,001-2 bedeuten,
wobei die Aktivierung durchgeführt werden kann durch Zusatz einer Halogenverbindung der Formel
R³-Z ,
worin
Z für Chlor, Brom oder Iod steht und
R³ Wasserstoff, Chlor, Brom oder Iod, den Rest eines anorganischen oder organischen Säurehalogenids, den Rest eines Benzyl-, Benzal-oder Benzotrihalogenids, C₄-C₈-tert.-Alkyl, Phenacyl, (Meth)Allyl oder -CHR¹-CHR²-OH, worin R¹ und R² die gegebene Bedeutung haben, bedeutet und für den Fall, daß Z für Iod steht, auch Cl₃ oder Br₃ bedeuten kann,
und bei einer Reaktionstemperatur von 40 bis 250°C, bevorzugt 50 bis 200°C, besonders bevorzugt 70 bis 170°C, und einem molaren Verhältnis von Alkylenoxid zu CO₂ von 1:1-10, bevorzugt 1:1-3, besonders bevorzugt 1:1-1,5, arbeitet.

2. Verfahren nach Anspruch 1, dadurch gekennzeichnet, daß zur Aktivierung eine Halogenverbindung der Formel
R¹³-Z
eingesetzt wird, in der
R¹³ Wasserstoff, C₄-C₆-tert.-Alkyl oder -CHR¹¹-CHR¹²-OH bedeutet, worin R¹¹ und R¹² unabhängig voneinander für H, CH₃, C₂H₅ oder Phenyl stehen, und
Z für Chlor, Brom oder Iod steht.

3. Verfahren nach Anspruch 1, dadurch gekennzeichnet, daß mindestens eines der Halogene X und Y Brom bedeutet, daß bevorzugt beide Halogene X und Y Brom bedeuten und daß besonders bevorzugt alle Halogene X, Y und Z Brom bedeuten.

4. Verfahren nach Anspruch 1, dadurch gekennzeichnet, daß die Aktivierung des Katalysators durch Zusatz der Halogenverbindung R³-Z vor seiner Rückführung zur erneuten Verwendung oder bereits vor seinem ersten Einsatz erfolgt.

5. Verfahren nach Anspruch 4, dadurch gekennzeichnet, daß die Aktivierung des Katalysators vor seinem ersten Einsatz und vor seiner Rückführung eine Halogenverbindung der Formel
R²³ - Br
eingesetzt wird, in der
R²³ Wasserstoff oder -CHR¹¹-CHR¹²-OH bedeutet, worin R¹¹ und R¹² unabhängig voneinander für H, CH₃, C₂H₅ oder Phenyl stehen.

6. Verfahren nach Anspruch 1, dadurch gekennzeichnet, daß für den Fall, daß als Halogenverbindung ein Halohydrin der Formel
Z - CHR¹ - CHR²- OH
eingesetzt wird, worin
R¹ und R² unabhängig voneinander Wasserstoff, substituiertes oder nicht substituiertes C₁-C₄-Alkyl, substituiertes oder nicht substituiertes C₂-C₄-Alkenyl oder substituiertes oder nicht substituiertes C₆-C₁₂-Aryl bedeuten,
dieses Halohydrin als solches eingesetzt wird oder als Vorstufe, aus der durch Zugabe von Halogenwasserstoff ein solches Halohydrin in situ erzeugt wird.

7. Verfahren nach Anspruch 1, dadurch gekennzeichnet, daß das Verhältnis der ganzen oder gebrochenen Zahlen a und b in einem Bereich für a:b von 20:1 bis 1:5, bevorzugt 10:1 bis 1:2, besonders bevorzugt von 3:1 bis 1:1, liegt.

8. Verfahren nach Anspruch 1, dadurch gekennzeichnet, daß man bei einem absoluten Reaktionsdruck von unter 30 bar, bevorzugt von unter 20 bar, besonders bevorzugt von unter 15 bar, arbeitet.

9. Verfahren nach Anspruch 1, dadurch gekennzeichnet, daß man nach der Umsetzung und vor oder nach der Abtrennung des gebildeten Alkylencarbonats die den Katalysator aktivierende Verbindung R³-Z in den gesamten Sumpf oder in einen Teil davon gibt, bei Zugabe von R³-Z vor der Abtrennung des gebildeten Alkylencarbonats diese Abtrennung vornimmt und die Sümpfe dann zurückführt.

10. Verfahren nach Anspruch 9, dadurch gekennzeichnet, daß für den Fall des Einsatzes einer aktivierenden Verbindung der Formel
R²³ - Br,
in der
R²³ Wasserstoff oder -CHR¹¹-CHR¹²-OH bedeutet, worin R¹¹ und R¹² unabhängig voneinander für H, CH₃, C₂H₅ oder Phenyl stehen,
bei der Aufarbeitung des Reaktionsgemisches vor dem Abdestillieren des Alkylencarbonats eine Leichtsiederfraktion mit der darin enthaltenen aktivierenden Verbindung R²³-Br entnimmt und diese Leichtsiederfraktion und den Destillationssumpf gemeinsam in die Reaktion zurückführt.

## Claims

1. Process for the preparation of alkylene carbonates of the formula 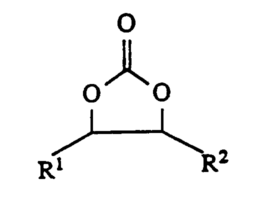 by reaction of alkylene oxides of the formula 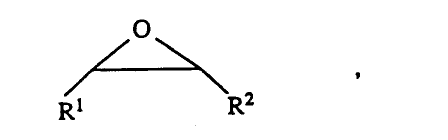 wherein, in the formulae,
R¹ and R² independently of one another denote hydrogen, substituted or unsubstituted C₁-C₄-alkyl, substituted or unsubstituted C₁-C₄-alkenyl or substituted or unsubstituted C₆-C₁₂-aryl and
R¹ and R², together with the two three-membered ring C atoms, can denote a saturated carbocyclic ring having 5-8 ring members,
with carbon dioxide in the presence of catalysts, characterised in that catalysts which can be activated, of the formula
a[MX]/b[ZnY₂]
wherein
M denotes an alkali metal, preferably Li, Na or K, particularly preferably Na or K,
X and Y independently of one another denote chlorine, bromine or iodine and
a and b denote fractions or integers in a range from 0.001 to 2,
are employed,
and the activation can be carried out by addition of a halogen compound of the formula
R³-Z
wherein
Z represents chlorine, bromine or iodine and
R³ denotes hydrogen, chlorine, bromine or iodine, the radical of an inorganic or organic acid halide, the radical of a benzyl, benzal or benzotrihalide, C₄-C₈-tert-alkyl, phenacyl, (meth)allyl or -CHR¹-CHR²-OH, in which R¹ and R² have the given meaning, and in the case where Z represents iodine, can also denote Cl₃ or Br₃,
and the process is carried out at a reaction temperature of 40 to 250°C, preferably 50 to 200°C, particularly preferably 70 to 170°C and at a molar ratio of alkylene oxide to CO₂ of 1:1-10, preferably 1:1-3, particularly preferably 1:1-1.5.

2. Process according to Claim 1, characterised in that a halogen compound of the formula
R¹³-Z
in which
R¹³ denotes hydrogen, C₄-C₆-tert-alkyl or -CHR¹¹-CHR¹²-OH, in which R¹¹ and R¹² independently of one another represent H, CH₃, C₂H₅ or phenyl, and
Z represents chlorine, bromine or iodine,
is employed for the activation.

3. Process according to Claim 1, characterised in that at least one of the halogens X and Y denotes bromine, in that preferably both halogens X and Y denote bromine, and in that particularly preferably all the halogens X, Y and Z denote bromine.

4. Process according to Claim 1, characterised in that the activation of the catalyst is carried out by addition of the halogen compound R³-Z prior to it being returned for re-use, or even before it is first used.

5. Process according to Claim 4, characterised in that, for the activation of the catalyst before it is first used and prior to it being returned, a halogen compound of the formula
R²³-Br
in which
R²³ denotes hydrogen or -CHR¹¹-CHR¹²-OH in which R¹¹ and R¹² independently of one another represent H, CH₃, C₂H₅ or phenyl
is employed.

6. Process according to Claim 1, characterised in that, when the halogen compound employed is a halohydrin of the formula
Z-CHR¹-CHR²-OH
in which
R¹ and R² independently of one another denote hydrogen, substituted or unsubstituted C₁-C₄-alkyl, substituted or unsubstituted C₂-C₄-alkenyl or substituted or unsubstituted C₆-C₁₂-aryl
this halohydrin is used as such or as a precursor from which such a halohydrin is generated in situ by addition of hydrogen halide.

7. Process according to Claim 1, characterised in that the ratio of the integers or fractions a and b is in a range for a:b of 20:1 to 1:5, preferably 10:1 to 1:2, particularly preferably 3:1 to 1:1.

8. Process according to Claim 1, characterised in that it is carried out under an absolute reaction pressure of less than 30 bar, preferably less than 20 bar, particularly preferably less than 15 bar.

9. Process according to Claim 1, characterised in that, after the reaction and before or after the removal of the alkylene carbonate formed, the compound R³-Z which activates the catalyst is added to the entire bottom product or to a part thereof, if R³-Z is added before the alkylene carbonate formed is separated off, this separation is undertaken, and the bottom products are then recycled.

10. Process according to Claim 9, characterised in that, when an activating compound of the formula
R²³-Br
in which
R²³ denotes hydrogen or -CHR¹¹-CHR¹²-OH in which R¹¹ and R¹² independently of one another represent H, CH₃, C₂H₅ or phenyl
is employed,
in the course of working up the reaction mixture, prior to removal by distillation of the alkylene carbonate, a low-boiling fraction with the activating compound R²³-Br present therein is removed and this low-boiling fraction is recycled to the reaction together with the bottom product from the distillation.

## Revendications

1. Procédé de préparation de carbonates d'alkylène de formule 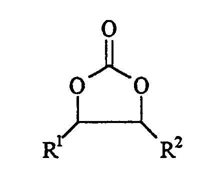 par réaction d'oxydes d'alkylène de formule 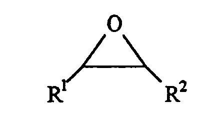 où, dans ces formules,
R¹ et R² représentent indépendamment l'un de l'autre un atome d'hydrogène ou un reste alkyle en C₁-C₄ substitué ou non substitué, alcényle en C₂-C₄ substitué ou non substitué ou aryle en C₆-C₁₂ substitué ou non substitué, et
R¹ et R² peuvent former avec les deux atomes de carbone du cycle à trois chaînons un noyau carbocyclique saturé de 5 à 8 chaînons,
avec du dioxyde de carbone en présence de catalyseurs,
caractérisé en ce que l'on utilise des catalyseurs susceptibles d'être activés de formule
a[MX]/b[ZnY₂]
dans laquelle
M représente un métal alcalin, de préférence Li, Na ou K, de façon particulièrement préférée Na ou K,
X et Y sont indépendamment l'un de l'autre un atome de chlore, de brome ou d'iode, et
a et b sont des nombres entiers ou fractionnaires compris entre 0,001 et 2,
l'activation pouvant être effectuée par addition d'un composé halogéné de formule
R³-Z,
dans laquelle
Z représente un atome de chlore, de brome ou d'iode, et
R³ représente un atome d'hydrogène, de chlore, de brome ou d'iode, le reste d'un halogénure d'acide inorganique ou organique, le reste d'un halogénure de benzyle, de benzylidène ou de benzényle, ou un reste tert-alkyle en C₄-C₈, phénacyle, (méth)allyle ou -CHR¹-CHR²-OH, où R¹ et R² ont la signification indiquée, et, dans le cas où Z est l'iode, R³ peut aussi être Cl₃ ou Br₃,
et en ce que l'on travaille à une température réactionnelle de 40 à 250°C, de préférence de 50 à 200°C, de façon particulièrement préférée de 70 à 170°C, et avec un rapport molaire de l'oxyde d'alkylène au CO₂ de 1:1-10, de préférence de 1:1-3, de façon particulièrement préférée de 1:1-1,5.

2. Procédé selon la revendication 1, caractérisé en ce que l'on utilise pour l'activation un composé halogéné de formule
R¹³-Z
dans laquelle
R¹³ représente un atome d'hydrogène ou un reste tert-alkyle en C₄-C₆ ou -CHR¹¹-CHR¹²-OH, où R¹¹ et R¹² sont indépendamment l'un de l'autre H, CH₃, C₂H₅ ou un phényle, et
Z représente un atome de chlore, de brome ou d'iode.

3. Procédé selon la revendication 1, caractérisé en ce qu'au moins l'un des halogènes X et Y représente du brome, en ce que de préférence les deux halogènes X et Y représentent le brome et en ce que, de façon particulièrement préférée, tous les halogènes X, Y et Z représentent le brome.

4. Procédé selon la revendication 1, caractérisé en ce que l'activation du catalyseur s'effectue par addition du composé halogéné R³-Z avant son recyclage pour une nouvelle utilisation ou déjà avant sa première utilisation.

5. Procédé selon la revendication 4, caractérisé en ce que l'on utilise pour activer le catalyseur, avant sa première utilisation ou avant son recyclage, un composé halogéné de formule
R²³-Br
dans laquelle
R²³ représente un atome d'hydrogène ou -CHR¹¹-CHR¹²-OH, où R¹¹ et R¹² sont indépendamment l'un de l'autre H, CH₃, C₂H₅ ou un phényle.

6. Procédé selon la revendication 1, caractérisé en ce que, dans le cas où on utilise comme composé halogéné une halogénhydrine de formule
Z - CHR¹ - CHR² - OH,
dans laquelle R¹ et R² représentent indépendamment l'un de l'autre un atome d'hydrogène ou un reste alkyle en C₁-C₄ substitué ou non substitué, alcényle en C₂-C₄ substitué ou non substitué ou aryle en C₆-C₁₂ substitué ou non substitué,
on utilise cette halogénhydrine telle quelle ou sous forme d'un produit intermédiaire à partir duquel on obtient une telle halogénhydrine *in situ* en ajoutant un halogénure d'hydrogène.

7. Procédé selon la revendication 1, caractérisé en ce que le rapport des nombres entiers ou fractionnaires a et b est compris dans un intervalle pour a:b de 20:1 à 1:5, de préférence de 10:1 à 1:2, de façon particulièrement préférée de 3:1 à 1:1.

8. Procédé selon la revendication 1, caractérisé en ce que l'on travaille sous une pression absolue de réaction inférieure à 30 bars, de préférence inférieure à 20 bars, de façon particulièrement préférée inférieure à 15 bars.

9. Procédé selon la revendication 1, caractérisé en ce que l'on ajoute le composé R³-Z activant le catalyseur dans tout ou partie du résidu après la réaction et avant ou après la séparation du carbonate d'alkylène formé, on procède à la séparation du carbonate d'alkylène formé dans le cas où on a ajouté R³-Z avant cette séparation, puis on recycle les résidus.

10. Procédé selon la revendication 9, caractérisé en ce que, dans le cas où on utilise un composé activateur de formule
R²³-Br
dans laquelle
R²³ représente un atome d'hydrogène ou -CHR¹¹-CHR¹²-OH, où R¹¹ et R¹² sont indépendamment l'un de l'autre H, CH₃, C₂H₅ ou un phényle,
lors du traitement du mélange réactionnel avant la distillation du carbonate d'alkylène formé, on prélève une fraction de produits de bas point d'ébullition contenant le composé activateur R²³-Br et on renvoie dans la réaction cette fraction de produits de bas point d'ébullition avec le résidu de la distillation.
